# EUROPÄISCHE PATENTSCHRIFT

(11) **EP 2 515 989 B1**
(45) Veröffentlichungstag und Bekanntmachung des Hinweises auf die Patenterteilung: **19.02.2014**
(21) Anmeldenummer: 10794872.1
(22) Anmeldetag: 07.12.2010
(51) Int. Cl.: A61M 27/00, A61M 1/00, A61F 13/02

(54) **ANSCHLUSSVORRICHTUNG ZUR VERWENDUNG BEI DER UNTERDRUCKBEHANDLUNG VON WUNDEN**
CONNECTING DEVICE FOR USE IN NEGATIVE PRESSURE WOUND THERAPY
DISPOSITIF DE RACCORDEMENT À UTILISER POUR LE TRAITEMENT SOUS DÉPRESSION DE PLAIES

(30) Priorität: 23.12.2009 DE 102009060596
(43) Veröffentlichungstag der Anmeldung: 31.10.2012
(73) Patentinhaber: Paul Hartmann Aktiengesellschaft, 89522 Heidenheim (DE)
(72) Erfinder: ECKSTEIN, Axel, 89522 Heidenheim (DE); HOFSTETTER, Jürgen, 89522 Heidenheim (DE); CROIZAT, Pierre, 89542 Herbrechtingen (DE); Klimenta Klaus, 38835 Osterwieck OT Zilly (DE); Süss Steffen, 38820 Halberstadt (DE)
(74) Vertreter: Dreiss
(86) Internationale Anmeldenummer: PCT/EP2010/007413
(87) Internationale Veröffentlichungsnummer: WO 2011/076340

(56) Entgegenhaltungen:
- EP-A1- 2 098 257
- WO-A1-03/057307
- WO-A1-2009/002260
- US-A1- 2009 234 307
- US-A1- 2009 281 526

## Beschreibung

Die Erfindung betrifft eine Anschlussvorrichtung zur Verwendung bei der Unterdruckbehandlung von Wunden, mit einem mit Unterdruck beaufschlagbaren Leitungsmittel, mit einem flächenhaften unterdruckdichten Trägermittel, an dessen wundabgewandter Oberseite das Leitungsmittel unterdruckdicht befestigt ist, wobei das Trägermittel auf einen die Wunde überfangenden und gegen die Atmosphäre dichtend abschließenden Unterdruckverband, vorzugsweise unter Verwendung einer zusätzlichen Klebefolie, aufbringbar ist, wobei das Leitungsmittel durch Öffnungen in dem Trägermittel und in dem Unterdruckverband hindurch mit dem Wundraum kommuniziert.

In der jüngeren Vergangenheit hat die Unterdruckbehandlung von Wunden, insbesondere von tiefen und daher a priori problematisch heilenden Wunden, eine zunehmende Bedeutung erlangt. Dabei bedeutet Unterdruckbehandlung, dass ein an sich der umgebenden Atmosphäre ausgesetzter Körper- oder Wundbereich durch noch näher zu beschreibende Mittel druckdicht bzw. unterdruckdicht gegen die Umgebung, also die Atmosphäre in der wir leben und atmen, abgeschlossen wird, wobei innerhalb des abgeschlossenen Wundbereichs auf ebenfalls noch zu erläuternde Weise ein gegenüber dem Atmosphärendruck verringerter Druck, mithin also Unterdruck relativ zur Atmosphäre angelegt und dauerhaft aufrechterhalten werden kann. Wenn auf dem hier in Rede stehenden Gebiet von Unterdruck die Rede ist, so wird hiermit ein Druckbereich verstanden, der typischerweise zwischen 0 und 500 mm Hg (mm Quecksilbersäule) unterhalb des umgebenden Atmosphärendrucks liegt. Es hat sich gezeigt, dass dies der Wundheilung förderlich ist. Für den unterdruckdichten Abschluss wird ein Unterdruckverband vorgesehen, der beispielsweise eine druck- bzw. unterdruckdichte Folienschicht umfassen kann, die typischerweise mit einem die Wunde umgebenden unversehrten Körperbereich verklebt ist, so dass auf diese Weise ein dichter Abschluss erreicht werden kann. Um ausgehend von einer Unterdruck erzeugenden Einrichtung, also einer Unterdruckpumpe im weitesten Sinn, einen Unterdruck an den Wundraum heranzuführen und dort aufrechtzuerhalten, können bei hier in Rede stehenden Systemen zur Unterdrucktherapie von Wunden mit Unterdruck beaufschlagbare Leitungsmittel verwendet werden, die mittels einer Anschlussvorrichtung mit dem Unterdruckverband zusammenwirken, um Unterdruck an bzw. in den Wundraum zu bringen. Eine derartige Anschlussvorrichtung der eingangs erwähnten Art ist beispielsweise aus WO 2006/052338 A2 bekannt. Es werden röhrenförmige Schläuche verwandt, um den Unterdruck an die Wunde heranzuführen. Eine weitere Anschlussvorrichtung wird in US 2009/281526 A1 offenbart.

Der vorliegenden Erfindung liegt die Aufgabe zugrunde, eine Anschlussvorrichtung der eingangs beschriebenen Art zu schaffen, die sich für den Patienten als angenehm bzw. wenig schmerzauslösend bei Belastung oder Berührung erweist und bei der die Abdichtung der Komponenten mit technisch und wirtschaftlich vertretbarem Aufwand sowie bedienerfreundlich realisierbar ist.

Diese Aufgabe wird bei einer Anschlussvorrichtung der genannten Art erfindungsgemäß dadurch gelöst, dass das Leitungsmittel biegsam und flach ausgebildet ist und die Breitenerstreckung des Leitungsmittels wenigstens 10 mm beträgt und das Leitungsmittel in einem wundseitigen Längsabschnitt mit wenigstens 70 % seiner senkrecht auf das Trägermittel projizierten Fläche mit dem Trägermittel für den bestimmungsgemäßen Gebrauch unlösbar und flächenhaft verbunden ist, und dass das Trägermittel und das Leitungsmittel im Bereich der miteinander unlösbar gefügten Flachseiten Öffnungen aufweisen, die einander überdecken also miteinander fluchten, und dass das Leitungsmittel aus einem biegsamen elastomeren Material einer Shore A - Härte von höchstens 60 gebildet ist und dass die Dickenerstreckung des Verbunds aus Leitungsmittel und Trägermittel höchstens 7 mm beträgt, und dass das Leitungsmittel im Inneren angeformte und mit dem Material des Leitungsmittels einstückig ausgebildete Mittel zur Verhinderung des Kollabierens des Leitungsmittels bei Unterdruckbeaufschlagung aufweist. Die Shore A - Härte wird bestimmt nach DIN 53505 vom August 2000. Die Dickenerstreckung des Verbunds aus Leitungsmittel und Trägermittel beträgt insbesondere höchstens 6 mm und weiter insbesondere höchstens 5 mm beträgt.

Das Leitungsmittel ist also nicht röhrenförmig mit im wesentlichen rundem Querschnitt ausgebildet, sondern es weist eine flache Form auf, dessen Breitenerstreckung wesentlich größer als seine Dickenerstreckung ist. Dies führt zusammen mit der Materialauswahl zu einem nachgiebigen Leitungsmittel, welches sich für den Patienten als angenehmer erweist, wenn auf die Anschlussvorrichtung oder das Leitungsmittel ein Berührungsdruck ausgeübt wird. Es kommt somit weniger zu einer punktuellen Belastung, die naturgemäß Schmerz auslösend sein kann und insbesondere bei schmerzempfindlichen frischen Wunden höchst problematisch ist. Außerdem besteht in Folge der flachen Ausbildung eine geringere Gefahr des Hinterhakens oder Hängenbleibens. Dadurch, dass das Leitungsmittel in demjenigen Längsabschnitt, mit dem es am Trägermittel befestigt ist, mit wenigstens 70 % seiner senkrecht auf das Trägermittel projizierten Fläche (wobei hierfür die Öffnung oder die Öffnungen in der Wandung des Leitungsmittels hinzugerechnet werden) mit dem flächenhaften Trägermittel flächenhaft verbunden ist, verteilt sich eine Druckbelastung auf einen großflächigeren Bereich des Wundverbands, was sich im Hinblick auf die vorstehend erörterte Problematik als sehr vorteilhaft auswirkt. Vorzugsweise ist das Leitungsmittel in dem der Befestigung dienenden Längsabschnitt mit wenigstens 80 %, insbesondere mit wenigstens 90 % und weiter insbesondere mit wenigstens 95 % seiner senkrecht das Trägermittel projizierten Fläche mit dem Trägermittel verbunden.

Die schon erwähnte Breitenerstreckung des flachen Leitungsmittels beträgt insbesondere wenigstens 15 mm und weiter insbesondere wenigstens 18 mm und insbesondere höchstens 30 mm und weiter insbesondere höchstens 25 mm.

Das elastomere Material, aus dem das Leitungsmittel gebildet ist, weist vorzugsweise eine Shore A - Härte von 5 - 60, insbesondere von 10 - 60, insbesondere von 15 - 50, insbesondere von 15 - 40 und weiter insbesondere von 15-35 auf. Wie schon vorausgehend erwähnt wird die Shore-A Härte nach DIN 53505 vom August 2000 bestimmt, und zwar bei 23°C an einem wie in der Norm beschrieben plattenförmigen ebenen und glatten Probekörper einer Dicke von 6 mm. Nach einer bevorzugten Ausführungsform der Erfindung ist das Leitungsmittel auf Silikonbasis ausgebildet.

Da das flache und biegsam ausgebildete Leitungsmittel zum Heranführen von Unterdruck in den Wundraum und gegebenenfalls zum Heranführen von Spülflüssigkeiten oder Spülgasen und dem Abführen von Wundsekreten dient, also vorzugsweise lediglich eine kanalbildende Kommunikationsfunktion hat, wird vorgeschlagen, das Leitungsmittel nicht laminatartig mit mehreren Komponenten oder Schichten auszubilden, sondern trotz seiner flachbauenden Form schlauchförmig, das heißt in einem Querschnitt betrachtet in Umfangsrichtung einstückig durchgehend aus einem einzigen Material auszubilden.

Weiter erweist es sich als vorteilhaft, dass das Leitungsmittel im Inneren angeformte, insbesondere mit dem Material des Leitungsmittels einstückig ausgebildete Mittel zur Verhinderung des Kollabierens des Leitungsmittels bei Unterdruckbeaufschlagung aufweist. Diese Mittel zur Verhinderung des Kollabierens des Leitungsmittel können gerade bei einem im vorstehend geschilderten Sinn schlauchförmigen Leitungsmittel vorgesehen werden. Diese Mittel zur Verhinderung des Kollabierens können beispielsweise von Rippen oder Vorsprüngen gebildet sein. In Weiterbildung dieses Erfindungsgedankens erweist es sich als vorteilhaft, wenn sie durchgehend erstreckt sind. Das Leitungsmittel kann dann in vorteilhafter Weise als Extrusionsteil ausgebildet sein.

Es kann sich ferner als vorteilhaft erweisen, wenn das Leitungsmittel mehrere voneinander druckdicht abgetrennte Kanäle umfasst, wobei das Leitungsmittel auch solchenfalls vorzugsweise einstückig ausgebildet ist, also keine Zusammenfassung von mehreren separaten kanalbildenden Mitteln aufweist. Die mehreren Kanäle können einen Spülkanal, der ein Spülmedium in Richtung auf das wundzugewandte Ende der Anschlussvorrichtung leiten kann, und einen Unterdruck führenden Kanal, der der Unterdruckzuführung bzw. der Abführung von Wundsekreten dient, umfassen. Hierdurch können auch etwaige Verstopfungen innerhalb des Leitungsmittels gelöst werden. Jeder Kanal kommuniziert dabei mit wenigstens einer Öffnung in dem Leitungsmittel.

Vorzugsweise erstreckt sich das flache Leitungsmittel über eine gewisse Distanz in Längsrichtung und kann dann über ein nicht dargestelltes Übergangs- oder Kopplungselement, welches eine Steckverbindung oder Klebeverbindung bilden kann, in einen üblichen verwindungssteiferen Rundschlauch übergehen, der zu einer Unterdruck erzeugenden Vorrichtung führt, die als stationäres Gerät oder als mobil am Körper des Patienten tragbares Gerät ausgebildet sein kann. Das Übergangs- oder Kopplungselement kann auch zum Koppeln eines mehrkanaligen Leitungsmittels mit einem mehrkanaligen Rundschlauch ausgebildet sein. Als zweckmäßige Längserstreckung des flachen Leitungsmittels hat sich eine Strecke von 10 - 60 cm erwiesen

Das flächenhafte Trägermittel der Anschlussvorrichtung, mit dem das flache Leitungsmittel herstellerseitig unterdruckdicht verbunden wird, ist vorzugsweise ebenfalls aus einem biegsamen elastomeren Material einer Shore A - Härte von 5 - 60, insbesondere von 10 - 60, insbesondere von 15 - 50, insbesondere von 15 - 40 und weiter insbesondere von 15 - 35 gebildet. Das flächenhafte Trägermittel weist vorteilhafterweise eine Dicke von 0,75 - 3 mm, insbesondere von 1 - 3 mm auf. Es ist nach einer bevorzugten Ausführungsform der Erfindung auf Silikonbasis ausgebildet. Nach einer weiteren bevorzugten Ausführungsform ist das Leitungsmittel und das Trägermittel aus demselben elastomeren Material ausgebildet. Das Trägermittel dient dazu, das flache Leitungsmittel in einem mit dem Wundraum kommunizierenden Längsendabschnitt zu halten und gleichmäßig zu stützen. Seine flächenhafte Erstreckung ist daher größer als die flächenhafte Erstreckung des Leitungsmittels in dem betreffenden wundseitigen Längsendabschnitt. Es erweist sich insoweit als vorteilhaft, wenn die flächenhafte Erstreckung des Trägermittels wenigstens das 1,5-fache vorzugsweise wenigstens das Doppelte der senkrecht auf das Trägermittel projizierten Fläche des Leitungsmittels umfasst, da hierdurch die über das Leitungsmittel bei Berührung eingeleiteten Kräfte über eine größere Fläche verteilt werden und auch Biegemomente, die auf das Leitungsmittel ausgeübt werden, nicht oder in geringerem Maße auf den Unterdruckverband übertragen werden; sie werden besser durch das plattenförmige Trägermittel aufgenommen. Es erweist sich als ausreichend, wenn das vorstehend erwähnte Flächenverhältnis höchstens 5, insbesondere höchstens 4 beträgt, wobei sich ein Verhältnis von 2 - 3 als vorteilhaft erwiesen hat.

Das flache Leitungsmittel könnte im Querschnitt betrachtet beispielsweise eine Rechteckform aufweisen, wobei die beiden Schmalseiten auch und vorzugsweise verrundet ausgebildet sein können. Nach einer weiteren Ausführungsform der Erfindung ist das Leitungsmittel im Querschnitt betrachtet trapezförmig ausgebildet. Die Schmalseiten fallen dann mit einem beispielhaften Neigungswinkel zur Ebene des flächenhaften Trägermittels von 25° bis 60°, insbesondere von 35° - 50° schräg ab, wobei die Flanken der einseitigen oder vorzugsweise zweiseitigen Trapezform nicht zwingend gerade, sondern durchaus auch verrundet verlaufen können.

Der unterdruckdichten Verbindung des flachen Leitungsmittels mit der wundabgewandten Oberseite des Trägermittels kommt eine funktional wesentliche Bedeutung zu. Es kommt hierfür grundsätzlich eine Klebeverbindung unter Verwendung eines Haftvermittlers im weitesten Sinn in Frage. Weiter erweist sich eine thermische Fügeverbindung als vorteilhaft, die einer Art Vulkanisierverbindung entsprechen kann. Beispielsweise kann das zuvor separat hergestellte Leitungsmittel auf das gerade frisch ausgegossene und nur teilweise erstarrte flächenhafte Trägermittel aufgebracht werden, so dass hierbei eine stoffschlüssige innige Anbindung der beiden Komponenten ohne Verwendung eines zusätzlichen Haftvermittlers erzielt wird.

Es erweist sich als vorteilhaft, wenn die miteinander kommunizierenden Öffnungen in dem Leitungsmittel und in dem Trägermittel einander überdecken, also miteinander fluchten. Dies erweist sich als am einfachsten herstellbar, wenn diese Öffnungen erst nach dem unterdruckdichten Verbinden des flachen Leitungsmittels mit dem flächenhaften Trägermittel gleichzeitig in beiden Komponenten ausgebildet werden. Dies kann beispielsweise durch einen materialherausformenden Stanzvorgang erreicht werden.

Im Hinblick auf die Anzahl und die Größe der Öffnungen in dem Leitungsmittel und in dem flächenhaften Trägermittel wäre es an sich denkbar, dass nur eine einzige Öffnung vorgesehen ist. Es erweist sich jedoch als vorteilhaft, wenn im unterdruckdichten Verbindungsbereich von Leitungsmittel und Trägermittel mehrere Öffnungen, insbesondere wenigstens zwei, insbesondere wenigstens vier Öffnungen je cm Länge des Leitungsmittels vorgesehen sind. Es erweist sich auch als vorteilhaft, wenn die lichte Öffnungsfläche der Öffnungen 5 - 50 % der Fläche der miteinander unlösbar gefügten Flachseiten von Leitungsmittel und Trägermittel beträgt.

Die erfindungsgemäße Anschlussvorrichtung umfasst also als Hauptkomponenten das flache und biegsam ausgebildete Leitungsmittel und das flächenhafte Trägermittel, welches das Leitungsmittel hält und gleichmäßig stützt. Um die Anschlussvorrichtung nun ihrerseits unterdruckdicht mit dem Unterdruckverband, welcher die zu behandelnde Wunde gegen die Atmosphäre dichtend abschließt, vorzugsweise lösbar zu verbinden, bedarf es weiterer Haftvermittler. Beispielsweise wäre es denkbar, wenn auf der dem Unterdruckverband zugewandten Unterseite des flächenhaften Trägermittels ein adhäsiv wirkendes Abdichtelement, beispielsweise eine Haftkleberbeschichtung oder eine Klebefolie vorgesehen ist. Nach einem weiteren Erfindungsgedanken erweist es sich als vorteilhaft, wenn das Trägermittel unter Verwendung einer zusätzlichen Klebefolie an dem Unterdruckverband festlegbar ist, wobei die zusätzliche Klebefolie auf der wundabgewandten Seite des flächenhaften Trägermittels vorgesehen ist und das Trägermittel entlang dessen Umrandung/Peripherie überragt, jedoch das Leitungsmittel vorzugsweise ausnimmt, also nicht überdeckt. Dadurch, dass das Leitungsmittel nicht überdeckt wird, werden in der Klebefolie keine Falten gebildet, die optisch und funktional im Hinblick auf die Abdichtungsfunktion problematisch wären. Mit der über die Peripherie des Trägermittels in Umfangsrichtung durchgehend überstehenden Bereich der Klebefolie kann dann die Anschlussvorrichtung auf die wundabgewandte Außenseite des Unterdruckverbands unterdruckdicht befestigt werden.

Dieser über die Peripherie überstehende Bereich der Klebefolie ist vorzugsweise mit einer abziehbaren Schicht überfangen, welche die Kleberbeschichtung vor dem Gebrauch der Anschlussvorrichtung konserviert, stützt und schützt.

Eine Stützwirkung kann in Weiterbildung der Erfindung auch dadurch vorgesehen werden, dass das Trägermittel oder die das Trägermittel peripher überfangende Klebefolie durch ein zusätzliches rahmenbildendes Stützmittel stabilisiert wird. Dieses rahmenbildende Stützmittel ist dann vorteilhafterweise auf der wundabgewandten Seite des Trägermittels oder der Klebefolie vorgesehen. Wenn es auf der Klebefolie vorgesehen ist, so ist es vorzugsweise außerhalb des Trägermittels vorgesehen. Es kann zum dauerhaften Verbleib an der Anschlussvorrichtung oder zum bestimmungsgemäßen Ablösen nach dem Applizieren der Anschlussvorrichtung auf dem Unterdruckverband ausgebildet sein.

Sämtliche vorausgehend beschriebenen Merkmale werden jeweils für sich und in beliebiger Kombination untereinander und mit weiteren Merkmalen als erfindungswesentlich betrachtet. Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der Erfindung.

In der Zeichnung zeigt:
- Figur 1: eine perspektivische Ansicht einer erfindungsgemäßen Anschlussvorrichtung zur Verwendung bei der Unterdruckbehandlung von Wunden;
- Figur 2: eine Draufsicht auf die Anschlussvorrichtung nach Figur 1;
- Figur 3: eine Ansicht der Anschlussvorrichtung nach Figur 1 von unten (Schutzfolie abgelöst);
- Figur 4: eine schematische nicht maßstabsgetreue Schnittansicht der Anschlussvorrichtung nach

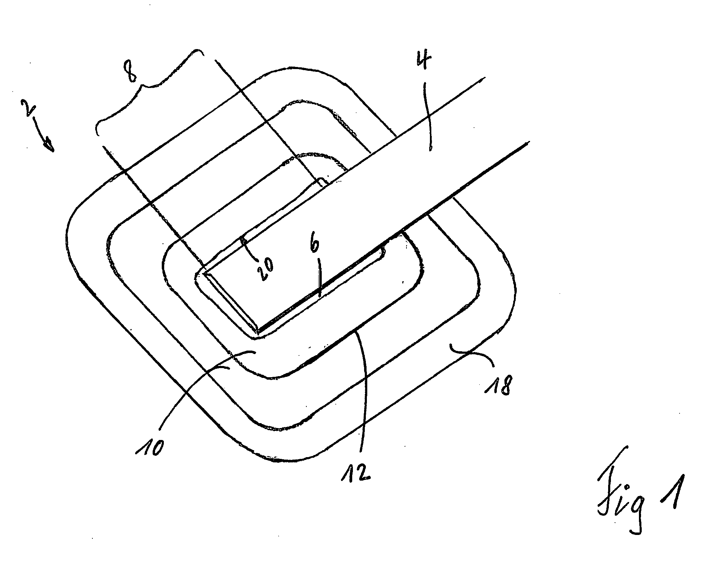

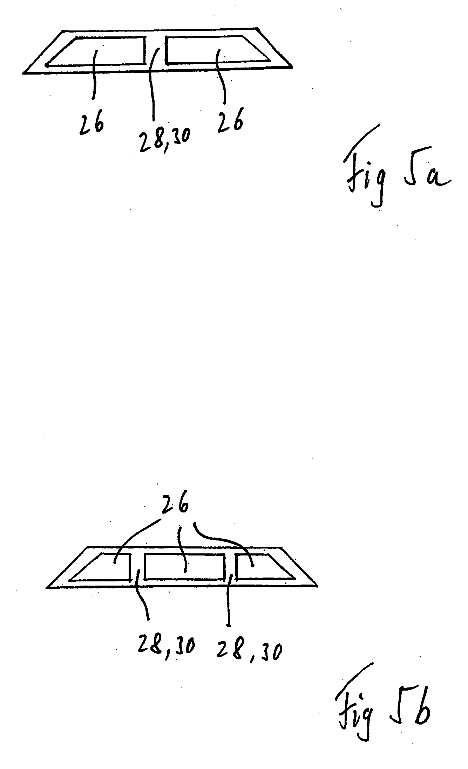

- Figur 1; Figuren 5a, b: Schnittansichten weiterer Ausführungsformen von Leitungsmitteln der erfindungsgemäßen Anschlussvorrichtung.

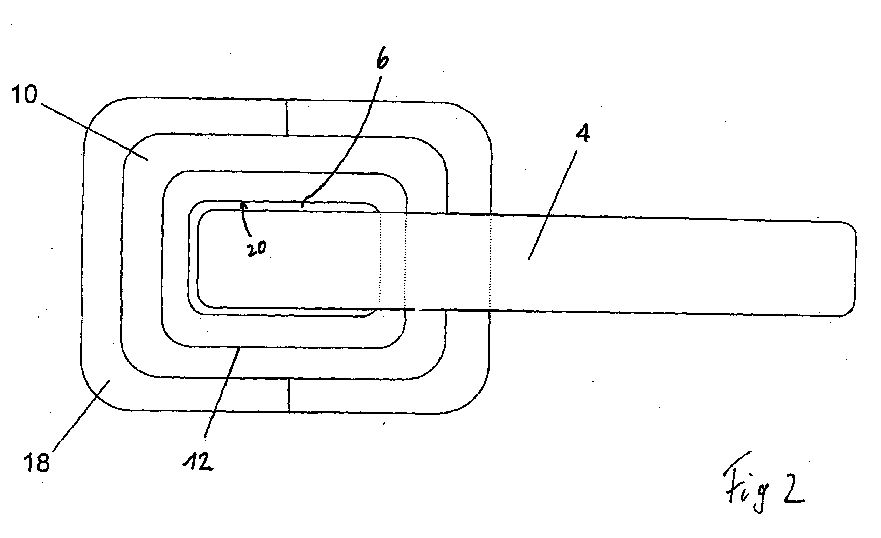

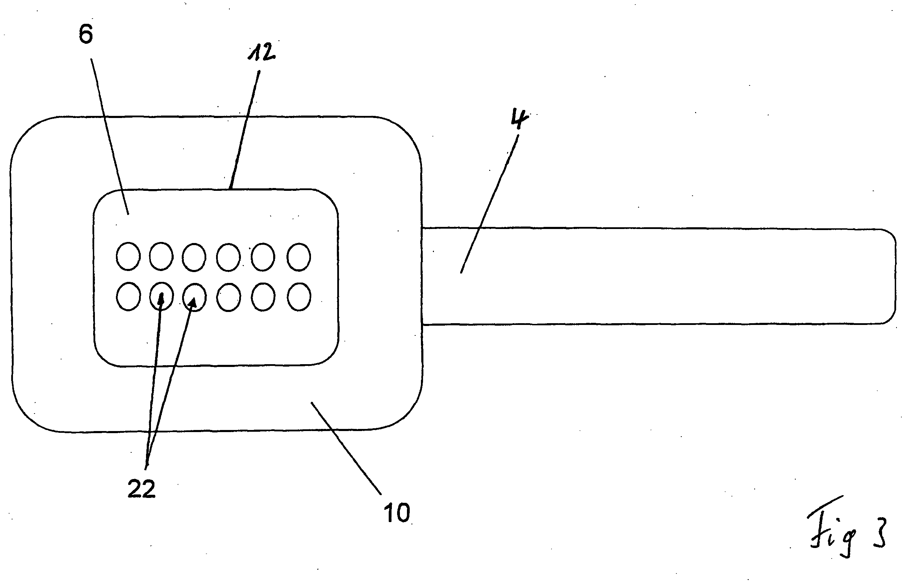

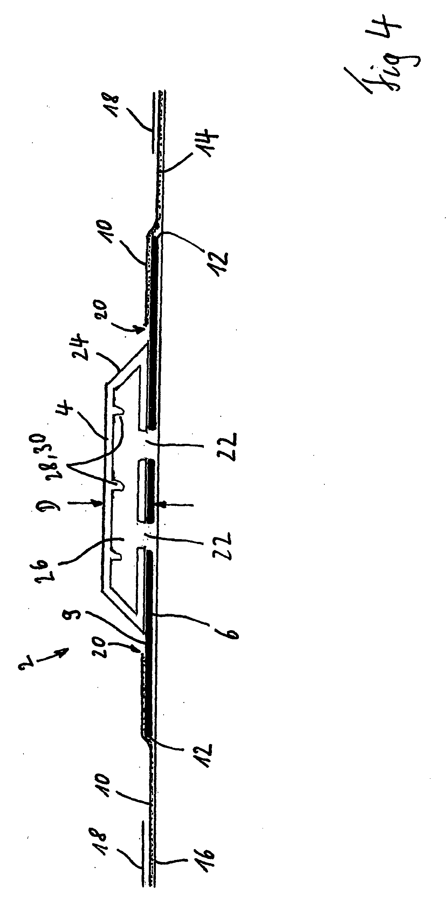

Die Figuren 1 - 4 zeigen verschiedene Ansichten einer erfindungsgemäßen insgesamt mit dem Bezugszeichen 2 bezeichneten Anschlussvorrichtung zur Verwendung bei der Unterdruckbehandlung von Wunden. Die dargestellte Anschlussvorrichtung 2 wird auf eine wundabgewandte Oberseite eines nicht dargestellten Unterdruckverbands, welcher eine zu behandelnde Wunde überfängt und zur Atmosphäre hin unterdruckdicht abschließt, vorzugsweise lösbar aufgebracht. Die Anschlussvorrichtung 2 umfasst ein flaches Leitungsmittel 4 aus einem elastomeren biegsamen Material und ein flächenhaft erstrecktes Trägermittel 6, welches das flache Leitungsmittel 4 hält und stützt, so dass auf das Leitungsmittel 4 ausgeübte Druck- oder Biegekräfte gleichmäßig in das Trägermittel 6 eingeleitet und von diesem aufgenommen werden können.

Das Leitungsmittel 4 ist derart flachbauend ausgebildet, dass es in einem Längsendabschnitt 8 mit nahezu 100 % seiner senkrecht auf das Trägermittel 6 projizierten Fläche mit dem Trägermittel 6 verbunden ist. Das Leitungsmittel 4 kann mit seiner betreffenden Flachseite auf die wundabgewandte Oberseite 9 des Trägermittels 6 aufgeklebt oder aufvulkanisiert, also thermisch gefügt sein.

Das flächenhafte plattenförmige Trägermittel 6 ist voll umfänglich von einer Klebefolie 10 überfangen, welche in Umfangsrichtung durchgehend über einen Umfangsrand 12 des Trägermittels 6 nach außen übersteht. Die Klebefolie 10 trägt eine Kleberbeschichtung 14, mittels derer die gesamte Anschlussvorrichtung 2 auf die wundabgewandte Außenseite eines nicht dargestellten Unterdruckverbands unterdruckdicht, jedoch vorzugsweise lösbar, aufbringbar ist. Der über den Umfangsrand 12 des Trägermittels 6 vorstehende Bereich der Kleberfolie 10 ist von einer abziehbaren Schutzfolie 16 von unten überdeckt. Auf der gegenüberliegenden Oberseite der Klebefolie 10 ist radial außen ein rahmenbildendes Stützmittel 18 vorgesehen. Die Klebefolie 10 weist eine innere Aussparung 20 auf, und zwar in demjenigen Bereich, in dem das Leitungsmittel 4 mit dem Trägermittel 6 verbunden ist, also im Bereich des Längsendabschnitts 8 des Leitungsmittels 4. Sofern technisch realisierbar, könnte die Klebefolie 10 aber auch das gesamte Trägermittel 6 überfangen, so dass sie zwischen dem Leitungsmittel 4 und dem Trägermittel 6 verläuft und in die Fügeverbindung zwischen Leitungsmittel 4 und Trägermittel 6 mit einbezogen ist.

Vorzugsweise nach dem unterdruckdichten Fügen des Leitungsmittels 4 mit dem Trägermittel 6 werden durch das Trägermittel 6 und durch das Leitungsmittel 4 hindurchgehende Öffnungen 22 ausgebildet, die mit nicht dargestellten Öffnungen in dem Unterdruckverband kommunizieren, wenn die Anschlussvorrichtung 2 auf den Unterdruckverband appliziert ist, um einen Unterdruck in den Wundraum anzulegen.

Das Leitungsmittel 4 ist in den Figuren trapezförmig ausgebildet und umfasst entlang seiner Längserstreckung zwei schräg in Richtung auf das Trägermittel abfallende Flanken 24, deren Winkel α zur Erstreckungsebene des Leitungsmittels 4 bzw. des Trägermittels 6 ca. 40 - 50° beträgt. Bei dem Leitungsmittel 4 gemäß Figuren 1 - 4 ist ein einziger Kanal 26 ausgebildet, der mit Unterdruck beaufschlagbar ist. Bei den alternativen Ausführungsformen nach Figuren 5a und 5b sind zwei bzw. drei Kanäle 26 ausgebildet, wobei der eine oder beide der äußeren kleineren Kanäle als Spülleitung zur Heranführung eines Spülmediums dienen können. Die Kanäle 26 kommunizieren an ihrem Ende in nicht dargestellter Weise miteinander.

Die Dickenerstreckung D des Verbunds aus Leitungsmittel 4 und Trägermittel 6 beträgt höchstens 7 mm, vorzugsweise höchstens 5 mm und weiter vorzugsweise lediglich 3 - 4 mm.

Das flachbauende und aus einem biegsamen Material, vorzugsweise auf Silikonbasis, und weiter vorzugsweise einstückig ausgebildete Leitungsmittel 4 ist an seiner Innenseite mit Mitteln 28 zur Verhinderung des Kollabierens des Leitungsmittels 4 ausgebildet. Diese Mittel 28 sind von in Längsrichtung durchgehenden Rippen 30 gebildet, die einstückig mit dem Leitungsmittel 4 hergestellt sind. Bei der Ausführungsform des Leitungsmittels 4 gemäß Figuren 5a und 5b werden diese Mittel 28 von den Wänden (Septen) zwischen den Kanälen 26 gebildet.

## Patentansprüche

1. Anschlussvorrichtung (2) zur Verwendung bei der Unterdruckbehandlung von Wunden, mit einem mit Unterdruck beaufschlagbaren Leitungsmittel (4), mit einem flächenhaften unterdruckdichten Trägermittel (6), an dessen wundabgewandter Oberseite (9) das Leitungsmittel (4) unterdruckdicht befestigt ist, wobei das Trägermittel (6) auf einen die Wunde überfangenden und gegen die Atmosphäre dichtend abschließenden Unterdruckverband, vorzugsweise unter Verwendung einer zusätzlichen Klebefolie (10), aufbringbar ist, wobei das Leitungsmittel (4) durch Öffnungen (22) in dem Leitungsmittel (4) und in dem Trägermittel (6) und in dem Unterdruckverband hindurch mit dem Wundraum kommuniziert, **dadurch gekennzeichnet, dass** das Leitungsmittel (4) biegsam und flach ausgebildet ist und die Breitenerstreckung des Leitungsmittels (4) wenigstens 10 mm beträgt und das Leitungsmittel (4) in einem wundseitigen Längsabschnitt (8) mit wenigstens 70 % seiner senkrecht auf das Trägermittel (6) projizierten Fläche mit dem Trägermittel (6) für den bestimmungsgemäßen Gebrauch unlösbar und flächenhaft verbunden ist, und dass das Trägermittel (6) und das Leitungsmittel (4) im Bereich der miteinander unlösbar gefügten Flachseiten Öffnungen (22) aufweisen, die einander überdecken also miteinander fluchten, und dass das Leitungsmittel (4) aus einem biegsamen elastomeren Material einer Shore A - Härte von höchstens 60 gebildet ist und dass die Dickenerstreckung (D) des Verbunds aus Leitungsmittel (4) und Trägermittel (6) höchstens 7 mm beträgt, und dass das Leitungsmittel (4) im Inneren angeformte und mit dem Material des Leitungsmittels einstückig ausgebildete Mittel (28) zur Verhinderung des Kollabierens des Leitungsmittels (4) bei Unterdruckbeaufschlagung aufweist.

2. Anschlussvorrichtung nach Anspruch 1, **dadurch gekennzeichnet, dass** das Leitungsmittel (4) in dem wundseitigen Längsabschnitt (8) mit wenigstens 80 %, insbesondere mit wenigstens 90 % und weiter insbesondere mit wenigstens 95 % seiner senkrecht auf das Trägermittel (6) projizierten Fläche mit dem Trägermittel (6) für den bestimmungsgemäßen Gebrauch unlösbar und flächenhaft verbunden ist.

3. Anschlussvorrichtung nach Anspruch 1 oder 2, **dadurch gekennzeichnet, dass** die Breitenerstreckung des Leitungsmittels (4) wenigstens 15 mm und weiter insbesondere wenigstens 18 mm und insbesondere höchstens 30 mm und weiter insbesondere höchstens 25 mm beträgt.

4. Anschlussvorrichtung nach Anspruch 1, 2 oder 3, **dadurch gekennzeichnet, dass** das Material, aus dem das Leitungsmittel (4) gefertigt ist, eine Shore A - Härte von 5 - 60, insbesondere von 10 - 60, insbesondere von 15 - 50, insbesondere von 15 - 40 und weiter insbesondere von 15 - 35 aufweist.

5. Anschlussvorrichtung nach einem oder mehreren der vorstehenden Ansprüche, **dadurch gekennzeichnet, dass** das Leitungsmittel (4) bei Betrachtung in einem Querschnitt in Umfangsrichtung schlauchförmig durchgehend ausgebildet ist.

6. Anschlussvorrichtung nach einem oder mehreren der vorstehenden Ansprüche, **dadurch gekennzeichnet, dass** die Mittel (28) zur Verhinderung des Kollabierens von Rippen (30) oder Vorsprüngen gebildet sind, die in Längsrichtung des Leitungsmittels (4) und insbesondere kontinuierlich durchgehend erstreckt sind.

7. Anschlussvorrichtung nach einem oder mehreren der vorstehenden Ansprüche, **dadurch gekennzeichnet, dass** das Leitungsmittel (4) mehrere voneinander druckdicht abgetrennte Kanäle (26) umfasst, wobei das Leitungsmittel (4) insbesondere einstückig ausgebildet ist.

8. Anschlussvorrichtung nach einem oder mehreren der vorstehenden Ansprüche, **dadurch gekennzeichnet, dass** das flächenhafte Trägermittel (6) aus einem biegsamen elastomeren Material einer Shore A - Härte von 5 - 60, insbesondere von 10 - 60, insbesondere von 15 - 50, insbesondere von 15 - 40 und weiter insbesondere von 15 - 35 gebildet ist und eine Dicke von vorzugsweise 0,75 - 3 mm, insbesondere 1 - 3 mm aufweist.

9. Anschlussvorrichtung nach einem oder mehreren der vorstehenden Ansprüche, **dadurch gekennzeichnet, dass** die flächenhafte Erstreckung des Trägermittels (6) wenigstens das 1,5-fache der senkrecht auf das Trägermittel (6) projizierten Fläche des Leitungsmittels (4) beträgt.

10. Anschlussvorrichtung nach einem oder mehreren der vorstehenden Ansprüche, **dadurch gekennzeichnet, dass** das Leitungsmittel (4) im Querschnitt betrachtet trapezförmig ausgebildet ist.

11. Anschlussvorrichtung nach einem oder mehreren der vorstehenden Ansprüche, **dadurch gekennzeichnet, dass** das Leitungsmittel (4) gegen die wundabgewandte Oberseite (9) des Trägermittels (6) geklebt oder thermisch angefügt ist.

12. Anschlussvorrichtung nach einem oder mehreren der vorstehenden Ansprüche, **dadurch gekennzeichnet, dass** im Verbindungsbereich von Leitungsmittel (4) und Trägermittel (6) wenigstens zwei Öffnungen (22) je cm Länge des Leitungsmittels (4) vorgesehen sind.

13. Anschlussvorrichtung nach einem oder mehreren der vorstehenden Ansprüche, **dadurch gekennzeichnet, dass** die lichte Öffnungsfläche der Öffnungen (22) 5 - 50 % der Fläche der miteinander unlösbar gefügten Flachseiten von Leitungsmittel (4) und Trägermittel (6) beträgt.

14. Anschlussvorrichtung nach einem oder mehreren der vorstehenden Ansprüche, **dadurch gekennzeichnet, dass** das flächenhafte Trägermittel (6) unter Verwendung einer zusätzlichen Klebefolie (10) an dem Unterdruckverband festlegbar ist, wobei die zusätzliche Klebefolie (10) auf der wundabgewandten Oberseite (9) des Trägermittels (6) vorgesehen ist und das Trägermittel (6) peripher überragt, jedoch das Leitungsmittel (4) vorzugsweise ausnimmt.

15. Anschlussvorrichtung nach einem oder mehreren der vorstehenden Ansprüche, **dadurch gekennzeichnet, dass** das Trägermittel (6) oder eine das Trägermittel (6) insbesondere peripher überfangende Klebefolie (10) ein peripher vorgesehenes rahmenbildendes Stützmittel (18) aufweist.

## Claims

1. Attachment device (2) for use in the vacuum therapy of wounds, with a conduit (4) to which a vacuum can be applied, with a large-surface vacuum tight mounting means (6), to whose topside (9), facing away from the wound, the conduit (4) is vacuum tightly fastened, wherein the mounting means (6) can be attached to a vacuum dressing which covers and tightly seals the wound against the atmosphere, preferably using an additional adhesive film (10), wherein the conduit (4) communicates with the wound space through openings (22) in the conduit (4) and in the mounting means (6) and in the vacuum dressing,
**characterized in that** the conduit (4) is constituted to be flexible and flat and that the width of the conduit (4) is at least 10 mm and that the conduit (4) is connected to the mounting means (6) for the intended use non-detachably and extensively in a longitudinal section (8) on the wound side over at least 70% of its surface projected perpendicularly onto the mounting means (6), and that, in the region of the flat sides that are non-detachably joined, the mounting means (6) and the conduit (4) have openings (22) that coincide, that is, are aligned with each other and that the conduit (4) is made from a flexible elastomer material of Shore A hardness of no more than 60, and that the thickness (D) of the combined conduit (4) and mounting means (6) being no more than 7 mm and that the conduit (4) in the interior has means (28) to prevent collapse of the conduit (4) when a vacuum is applied that are integrally molded forming one integral element with the material of the conduit.

2. Attachment device according to claim 1, **characterized in that** the conduit (4) is connected to the mounting means (6) for the intended use, non-detachably and extensively in the longitudinal section (8) on the wound side over at least 80%, in particular, at least 90% and further, in particular, over at least 95% of its surface projected perpendicularly onto the mounting means (6).

3. Attachment device according to either one of the claims 1 or 2, **characterized in that** the width of the conduit (4) is at least 15 mm and further, in particular, at least 18 mm and, particular, no more than 30 mm and further, in particular, no more than 25 mm.

4. Attachment device according to any one of the claims 1, 2 or 3, **characterized in that** the material from which the conduit (4) is made has a Shore A hardness of 5 to 60, in particular, of 10 to 60, in particular, of 15 to 50, in particular, of 15 to 40 and further in particular, of 15 to 35.

5. Attachment device according to one or more of the previous claims, **characterized in that** the conduit (4), seen in cross-section in the circumferential direction, is completely tubular.

6. Attachment device according to one or more of the preceding claims, **characterized in that** that means (28) to prevent collapse are formed from ribs (30) or protrusions, which extend in the longitudinal direction of the conduit (4), in particular, continuously from end to end.

7. Attachment device according to one or more of the previous claims, **characterized in that** the conduit (4) comprises multiple ducts (26) pressure-tightly separated from each other, wherein the conduit (4) is preferably constituted as one integral element.

8. Attachment device according to one or more of the previous claims, **characterized in that** the large-area mounting means (6) is made of a flexible elastomer material with a Shore A hardness of 5 to 60, in particular, of 10 to 60, in particular, of 15 to 50, in particular, of 15 to 40 and further, in particular, of 15 to 35 and has a thickness of preferably 0.75 to 3 mm, in particular, 1 to 3 mm.

9. Attachment device according to one or more of the previous claims, **characterized in that**, projected perpendicularly onto the mounting means (6), the area of the mounting means (6) is at least 1.5 times the area of the conduit (4).

10. Attachment device according to one or more of the previous claims, **characterized in that**, seen in cross-section, the conduit (4) is trapezoidal in shape.

11. Attachment device according to one or more of the previous claims, **characterized in that** the conduit (4) is adhesively bonded or thermally joined to the topside (9) of the mounting means (6) that is facing away from the wound.

12. Attachment device according to one or more of the previous claims, **characterized in that**, in the connection region of the conduit (4) and mounting means (6), at least two openings (22) per cm length of the conduit (4) are provided.

13. Attachment device according to one or more of the previous claims, **characterized in that** the clearance opening area of the openings (22) is 5 to 50 % of the area of the non-detachably joined flat sides of the conduit (4) and mounting means (6).

14. Attachment device according to one or more of the previous claims, **characterized in that** the large-area mounting means (6) can be attached to the vacuum dressing using an additional adhesive film (10), wherein the additional adhesive film (10) is provided on the topside of the mounting means (6) facing away from the wound and overlaps the mounting means (6) along its perimeter but preferably omits the conduit (4).

15. Attachment device according to one or more of the previous claims, **characterized in that** the mounting means (6) or the adhesive film overlapping the mounting means, in particular at the perimeter, has a peripheral frame-forming support means (18).

## Revendications

1. Dispositif de raccordement (2) utilisé pour le traitement par pression négative de plaies, comprenant un moyen de conduite (4) apte à être soumis à une pression négative, un moyen support (6) en nappe qui est étanche à la pression négative et sur la surface supérieure (9) duquel, qui est opposée à la plaie, ledit moyen de conduite (4) est fixé d'une manière étanche à la pression négative, ledit moyen support (6) pouvant être appliqué sur un pansement à pression négative recouvrant la plaie et fermant celle-ci de façon étanche par rapport à l'atmosphère, de préférence en utilisant une feuille adhésive (10) supplémentaire, ledit moyen de conduite (4) communiquant via des ouvertures (22) ménagées dans le moyen de conduite (4) et dans ledit moyen support (6) et dans ledit pansement à pression négative avec l'espace de la plaie, **caractérisé par le fait que** ledit moyen de conduite (4) est réalisé de manière flexible et plate et l'extension en largeur du moyen de conduite (4) est de 10 mm au moins et que ledit moyen de conduite (4) est, dans une portion longitudinale (8) côté plaie, relié au moyen support (6) d'une manière non détachable et en nappe, pour l'utilisation conforme à la destination, par au moins 70 % de sa surface projetée perpendiculairement sur ledit moyen support (6), et que le moyen support (6) et le moyen de conduite (4) présentent, au niveau des faces plates jointes entre elles de façon non détachable, des ouvertures (22) qui se recouvrent les unes les autres, donc sont alignées, et que ledit moyen de conduite (4) est réalisé dans une matière flexible élastomère ayant une dureté Shore A de 60 tout au plus et que l'extension en épaisseur (D) de l'ensemble constitué par le moyen de conduite (4) et le moyen support (6) est de 7 mm tout au plus, et que le moyen de conduite (4) présente des moyens (28) qui sont surmoulés à l'intérieur et réalisés d'un seul tenant avec la matière du moyen de conduite et destinés à éviter que le moyen de conduite (4) ne subisse un collapsus lorsqu'il est soumis à la pression négative.

2. Dispositif de raccordement selon la revendication 1, **caractérisé par le fait que** le moyen de conduite (4) est, dans ladite portion longitudinale (8) côté plaie, relié au moyen support (6) d'une manière non détachable et en nappe, pour l'utilisation conforme à la destination, par au moins 80 %, en particulier par au moins 90 % et encore en particulier par au moins 95 % de sa surface projetée perpendiculairement sur ledit moyen support (6).

3. Dispositif de raccordement selon la revendication 1 ou 2, **caractérisé par le fait que** l'extension en largeur du moyen de conduite (4) est de 15 mm au moins et encore en particulier de 18 mm au moins et en particulier de 30 mm tout au plus et encore en particulier de 25 mm tout au plus.

4. Dispositif de raccordement selon la revendication 1, 2 ou 3, **caractérisé par le fait que** la matière à partir de laquelle le moyen de conduite (4) est réalisé présente une dureté Shore A comprise entre 5 et 60, en particulier entre 10 et 60, en particulier entre 15 et 50, en particulier entre 15 et 40 et encore en particulier entre 15 et 35.

5. Dispositif de raccordement selon l'une ou plusieurs des revendications précédentes, **caractérisé par le fait que** ledit moyen de conduite (4) est réalisé, vu dans une coupe transversale, dans la direction circonférentielle, de part en part en forme de tuyaux flexible.

6. Dispositif de raccordement selon l'une ou plusieurs des revendications précédentes, **caractérisé par le fait que** lesdits moyens (28) destinés à éviter un collapsus sont constitués par des nervures (30) ou projections qui s'étendent dans la direction longitudinale du moyen de conduite (4) et, en particulier, de façon continue de part en part.

7. Dispositif de raccordement selon l'une ou plusieurs des revendications précédentes, **caractérisé par le fait que** ledit moyen de conduite (4) comprend une pluralité de canaux (26) isolés les un des autres d'une manière étanche à la pression, ledit moyen de conduite (4) étant réalisé de préférence en une seule pièce.

8. Dispositif de raccordement selon l'une ou plusieurs des revendications précédentes, **caractérisé par le fait que** ledit moyen support (6) en nappe est réalisé dans une matière flexible élastomère ayant une dureté Shore A comprise entre 5 et 60, en particulier entre 10 et 60, en particulier entre 15 et 50, en particulier entre 15 et 40 et encore en particulier entre 15 et 35, et présente une épaisseur comprise de préférence entre 0,75 et 3 mm, en particulier entre 1 et 3 mm.

9. Dispositif de raccordement selon l'une ou plusieurs des revendications précédentes, **caractérisé par le fait que** l'extension en nappe du moyen support (6) est au moins 1,5 fois la surface du moyen de conduite (4) projetée perpendiculairement sur le moyen support (6).

10. Dispositif de raccordement selon l'une ou plusieurs des revendications précédentes, **caractérisé par le fait que**, vu en coupe transversale, ledit moyen de conduite (4) est trapézoïdal.

11. Dispositif de raccordement selon l'une ou plusieurs des revendications précédentes, **caractérisé par le fait que** ledit moyen de conduite (4) est collé ou joint thermiquement contre la surface supérieure (9) du moyen support (6) qui est opposée à la plaie.

12. Dispositif de raccordement selon l'une ou plusieurs des revendications précédentes, **caractérisé par le fait que** dans la zone de jonction du moyen de conduite (4) et du moyen support (6) sont prévues au moins deux ouvertures (22) par centimètre de longueur du moyen de conduite (4).

13. Dispositif de raccordement selon l'une ou plusieurs des revendications précédentes, **caractérisé par le fait que** la surface libre d'ouverture des ouvertures (22) est comprise entre 5 et 50 % de la surface des faces plates des moyens de conduite (4) et support (6) jointes entre elles de manière non détachable.

14. Dispositif de raccordement selon l'une ou plusieurs des revendications précédentes, **caractérisé par le fait que** ledit moyen support (6) en nappe peut être fixé sur le pansement à pression négative en utilisant une feuille adhésive (10) supplémentaire, ladite feuille adhésive (10) supplémentaire étant prévue sur la surface supérieure (9) du moyen support (6) qui est opposée à la plaie et dépassant le moyen support (6) sur la périphérie tout en excluant cependant, de préférence, le moyen de conduite (4).

15. Dispositif de raccordement selon l'une ou plusieurs des revendications précédentes, **caractérisé par le fait que** ledit moyen support (6) ou une feuille adhésive (10) s'étendant sur le moyen support (6) en particulier sur la périphérie présente un moyen d'appui (18) formant cadre prévu sur la périphérie.
